# FASCICULE DE BREVET EUROPEEN

(11) **EP 0 697 206 B1**
(45) Date de publication et mention de la délivrance du brevet: **31.07.1996**
(21) Numéro de dépôt: 95400286.1
(22) Date de dépôt: 10.02.1995
(51) Int. Cl.: A61K 7/00, A61K 7/50

(54) **Microémulsion cosmétique et/ou dermatologique, ses utilisations**
Kosmetische oder dermatologische Mikroemulsion und ihre Verwendung
Cosmetic or pharmaceutical microemulsion and uses thereof

(30) Priorité: 11.03.1994 FR 9402881
(43) Date de publication de la demande: 21.02.1996
(73) Titulaire: L'OREAL, F-75008 Paris (FR)
(72) Inventeur: de Salvert, Armelle, F-75013 Paris (FR); Fodor, Pierre, F-92380 Garches (FR); Pouget, Françoise, F-94120 Fontenay-Sous-Bois (FR)
(74) Mandataire: Dodin, Catherine

(56) Documents cités:
- EP-A- 0 334 777
- EP-A- 0 516 508
- WO-A-93/12766

## Description

La présente invention se rapporte à une composition cosmétique et/ou dermatologique sous forme d'une microémulsion dans laquelle le système émulsionnant comprend le mélange d'un émulsionnant et d'un coémulsionnant. L'invention a pour objet également l'utilisation de cette microémulsion et un procédé pour traiter la peau, y compris le cuir chevelu, les ongles et/ou les cheveux. La microémulsion peut être aussi bien utilisée sur le visage que sur le corps par application topique.

Une microémulsion est un système dispersé obtenu à partir de quatre composants initiaux : une phase aqueuse, une phase huileuse, un émulsionnant et un coémulsionnant. La composition quaternaire obtenue conduit à une microstructure particulière formée de microgouttelettes dont la taille est telle que la lumière les traverse sans être diffusée, si bien que l'aspect de cette composition est transparente alors que l'aspect d'une émulsion est opaque.

Par rapport à une émulsion, une microémulsion présente l'avantage de permettre une meilleure pénétration des actifs du fait de la petite taille de ses microgouttelettes et d'être donc un meilleur vecteur pour les actifs. Elle a aussi l'avantage d'être préparée à froid, ce qui permet d'une part de diminuer la consommation d'énergie lors de sa fabrication et, d'autre part, d'utiliser des actifs thermosensibles qu'il était impossible d'utiliser dans une émulsion sous peine de les dégrader. Un troisième avantage d'une microémulsion réside dans le fait qu'elle peut contenir une plus grande quantité d'actifs difficiles à solubiliser du fait d'une stabilité beaucoup plus grande que celle des émulsions, dûe notamment à une quantité plus importante d'émulsionnants (25 % à 50 % en poids par rapport au poids total de la microémulsion au lieu de 3 % à 5 % dans une émulsion).

En revanche, cette quantité plus importante d'émulsionnant présente des inconvénients et peut entraîner des irritations, voire des picotements, ce qui explique que l'on utilise plus fréquemment des émulsions que des microémulsions dans les domaines cosmétique et/ou dermatologique.

Aussi, on cherche depuis longtemps à diminuer l'effet irritant des microémulsions, notamment dans les domaines pharmaceutique et cosmétique. A cet effet, le brevet FR2628632 envisage l'utilisation d'un mélange particulier d'émulsionnant et de coémulsionnant pour obtenir une microémulsion à usage cosmétique ou pharmaceutique présentant une bonne innocuité. L'émulsionnant et le coémulsionnant sont constitués, respectivement, d'un coester de polyéthylèneglycol et de glycérol avec un acide gras comme les glycérides caprylate / caprate de polyéthylèneglycol 400, et d'un ester de polyglycérol avec un acide gras comme l'isostéarate de polyglycéryl-6. Néanmoins, les microémulsions exemplifiées dans ce brevet ont l'inconvénient d'être peu confortables à l'application sur la peau.

De plus, ces microémulsions ont un caractère collant et poisseux qui est désagréable pour l'utilisateur, ainsi qu'un goût amer qui peut rendre leur utilisation rédhibitoire pour une application sur le visage, à proximité des lèvres, et/ou sur les doigts. Un panel de plusieurs personnes a constaté unanimement le manque de confort et le goût amer de telles compositions.

Il subsiste donc le besoin d'une microémulsion cosmétiquement agréable à utiliser, non amère et peu irritante.

La microémulsion selon l'invention permet justement de pallier les problèmes mentionnés précédemment. En effet, la demanderesse a trouvé de manière surprenante qu'il était possible d'obtenir une microémulsion ayant de bonnes propriétés cosmétiques en utilisant un émulsionnant particulier et une phase huileuse en une quantité plus grande que celle utilisée dans l'art antérieur.

La présente invention a donc pour objet une microémulsion cosmétique et/ou dermatologique comportant comme émulsionnant un coester de polyéthylèneglycol et de glycérol avec un acide gras et comme coémulsionnant un ester de polyglycérol avec un acide gras, caractérisée en ce qu'elle comprend au moins 30 % en poids d'huile par rapport au poids total de la composition, et en ce que le coester est un laurate de polyéthylèneglycol/laurate de glycérol.

Le choix particulier de l'émulsionnant permet de supprimer l'amertume de la microémulsion. De plus, la microémulsion de l'invention, du fait de sa quantité d'huile élevée, ne présente plus d'aspect collant et est confortable à l'application. Or, rien ne permettait de conduire l'homme de métier à augmenter la phase huileuse pour diminuer l'effet collant.

Certes, il est connu du document EP-A-516508 des microémulsions contenant un émulsionnant et un coémulsionnant constitués, respectivement, d'un coester de polyéthylèneglycol et de glycérol avec un acide gras, et d'un ester de polyglycérol avec un acide gras comme l'isostéarate de polyglycéryl-6. Le coester de polyéthylèneglycol et de glycérol utilisé comme émulsionnant y est un oléate ou un isostéarate. Le laurate n'est ni cité ni exemplifié dans ce document.

Les diagrammes décrits plus loin montrent qu'en utilisant un laurate comme coester, on peut obtenir une microémulsion dans une large zone alors qu'avec un autre ester d'acide gras, et dans le cas présent l'isostéarate, il n'est pas possible d'obtenir de microémulsion. Ainsi, dans le document EP-A-516508, pour obtenir une microémulsion stable, il est nécessaire d'ajouter un éther phosphate (voir colonne 4, lignes 2 à 6). L'utilisation d'un laurate dans la composition selon l'invention ne nécessite pas la présence de cet éther phosphate pour obtenir une microémulsion stable, même avec des taux d'huile importants. Le document EP-A-516508 ne conduit nullement l'homme du métier à utiliser le laurate seul comme coester émulsionnant pour obtenir une microémulsion stable.

Les microémulsions objet de l'invention trouvent leur application dans un grand nombre de traitements cosmétiques et/ou dermatologiques de la peau, y compris le cuir chevelu et les ongles, comme par exemple l'hydratation, la nutrition, la protection, le raffermissement, le traitement du vieillissement, le traitement des rides et/ou ridules, l'amincissement, la dépigmentation, le traitement de l'acné, et les traitements des mycoses, des dermites et du psoriasis.

Aussi, l'invention a encore pour objet une utilisation de la microémulsion définie ci-dessus pour le traitement cosmétique de la peau, du visage et/ou du corps, des cheveux et des ongles, notamment pour hydrater, nourrir, protéger, raffermir, éliminer les rides et/ou les ridules et/ou l'acné, amincir et/ou dépigmenter, ainsi que pour la préparation d'une composition destinée au traitement des maladies de la peau et/ou des ongles, et notamment des mycoses, des dermites et du psoriasis.

L'invention a encore pour objet un procédé de traitement cosmétique de la peau, des ongles et/ou des cheveux consistant à appliquer la microémulsion ci-dessus sur la peau, les ongles et/ou les cheveux.

Dans la suite du texte, on utilisera la nomenclature CTFA.

Dans les microémulsions selon l'invention, la phase huileuse comprend au moins une huile animale, une huile végétale, une huile minérale, une huile siliconée (par exemple une diméthicone), une huile fluorée et/ou une huile synthétique. On peut citer notamment l'isotéarate d'isostéaryle, le PPG-15 stéaryl éther, l'isohexadécane, le monococoate d'éthyl-2 hexyle et le palmitate d'isopropyle. La phase huileuse peut comprendre aussi des extraits huileux comme l'oléat d'origan. La phase huileuse représente jusqu'à 75 % en poids par rapport au poids total de la microémulsion, notamment de 25 à 75 % et de préférence de 50 % à 70 % en poids par rapport au poids total de la microémulsion.

Le laurate de polyéthylèneglycol / laurate de glycérol est de préférence le laurate de polyéthylèneglycol-400 / laurate de glycérol. Le taux d'émulsionnant peut aller de 10 % à 60 % en poids par rapport au poids total de la microémulsion et, de préférence de 12 % à 30 % en poids par rapport au poids total de la microémulsion.

L'ester de polyglycérol avec un acide gras est de préférence un isostéarate de polyglycéryle ou un laurate de polyglycéryle, par exemple l'isostéarate ou le laurate de polyglycéryle-6 contenant 6 molécules de glycérine. Le taux de coémulsionnant peut aller de 4 % à 40 % en poids par rapport au poids total de la microémulsion, et de préférence de 6 % à 15 % en poids par rapport au poids total de la microémulsion.

Le taux total de l'émulsionnant et du co-émulsionnant peut aller de 20 % à 80 % en poids, et de préférence de 25 % à 35 % en poids par rapport au poids total de la microémulsion. Le rapport de l'émulsionnant au co-émulsionnant peut aller de 1 à 4.

De façon connue, les microémulsions cosmétiques ou dermatologiques de l'invention peuvent, en outre, contenir des adjuvants habituels dans le domaine cosmétique tels que les gélifiants hydrophiles ou lipophiles, les conservateurs, les antioxydants, les parfums, les charges, les filtres, les matières colorantes, les actifs hydrophiles ou lipophiles, et encore des vésicules lipidiques.

Les actifs pour la peau peuvent être des actifs anti-âge, des actifs anti-rides, des hydratants ou des humectants, des actifs amincissants, des actifs dépigmentants, des actifs anti-radicaux libres (espèces radicalaires de l'oxygène), des actifs nutritifs, des actifs protecteurs, des actifs restructurants, des actifs raffermissants, des actifs antiacnéiques, des actifs exfoliants, des actifs émollients ou encore des actifs traitants des maladies de peau comme les mycoses, les dermites, le psoriasis, etc. Ces actifs sont utilisés, selon leur nature, dans les proportions habituelles des microémulsions, et par exemple de 0,01 % à 10 % en poids par rapport au poids total de la microémulsion.

Comme actifs anti-âge et/ou anti-rides, on peut citer les agents kératolytiques tels que les hydroxyacides (acide lactique, acide glycolique, acide salicylique et ses dérivés), le rétinol (vitamine A) et ses dérivés, les sucres et leurs dérivés, les protides, les céramides, les acides gras essentiels, le farnesol, ses dérivés et les mélanges les contenant tels que le mélange d'acétate de farnesyle, de farnesol et de triacétate de panthenyle vendu sous la dénomination Unitrienol T-27 par la société Lipo, les phospholipides extraits de poulpe, riches en oligo-éléments et en acides gras polyinsaturés, les glycosphingolipides et les mélanges les contenant tels que celui vendu sous la dénomination AFR LS par la société des Laboratoires Sériobiologiques.

Comme hydratants ou humectants, on peut citer la glycérine et les autres polyols tels que le sorbitol, I'urée, les aminoacides et leurs dérivés tels que l'hydroxyproline, les protéines et les hydrolysats de protéine, I'allantoïne et les mélanges complexes tels que celui vendu sous le nom d'Hydroviton par la société Dragoco.

Comme amincissants, on peut citer par exemple les agents liporégulateurs tels que la caféine, la théophylline, le centella asiatica, l'acide asiatique et ses dérivés, le mélange d'enzyme, de sulfosaccharide et de sels biliaires, vendu sous la dénomination Biorubine par la société Schmidt-Jourdan, ou le mélange vendu sous la dénomination Fibrastil par la société Sederma.

Comme dépigmentants, on peut citer l'acide caféique, l'acide kojique, l'acide ascorbique (vitamine C) et ses dérivés, et l'hydroquinone.

Comme anti-radicaux libres, on peut citer le tocophérol (vitamine E) et ses dérivés, et la superoxyde dismutase.

Comme actifs nutritifs, restructurants et raffermissants, on peut citer les sucres, les acides aminés, les acides gras, les vitamines, les protéines telles que le collagène, notamment le collagène marin, le mélange de parléam, d'acides gras polyinsaturés, d'extrait d'algues vendu sous la dénomination Recopier System Lipo par la société Sederma, et l'acide salicylique et ses dérivés tels que le salicylate d'isodécyle vendu sous la dénomination Keratoplast par la société Vevy.

Comme actifs protecteurs, on peut citer les filtres solaires, les acides aminés et la dihydroxyacétone.

Comme actifs anti-acnéiques, on peut citer la trétinoïne et l'isotrétinoïne (vitamine A acide), l'octopirox, l'acide salicylique et ses dérivés, les α-hydroxy-acides , le peroxyde de benzoyle.

Les actifs ayant une application capillaire peuvent être des actifs stimulant la pousse des cheveux tels que le minoxidil.

Les actifs pour traiter les maladies de la peau peuvent être des exfoliants, des antimycotiques, des antiinflammatoires.

Comme gélifiants hydrophiles, on peut citer les polymères carboxyvinyliques, les gommes naturelles et les argiles, et, comme gélifiants lipophiles, on peut citer les argiles modifiées comme les bentones et les dérivés de silice tels que les silices vendues sous la dénomination d'Aérosil par la société Degussa.

On donne ci-après des exemples des microémulsions selon l'invention à titre illustratif et non limitatif. Toutes les microémulsions sont préparées par mélange des constituants à froid sous agitation planétaire. Les pourcentages des compositions sont des pourcentages en poids.

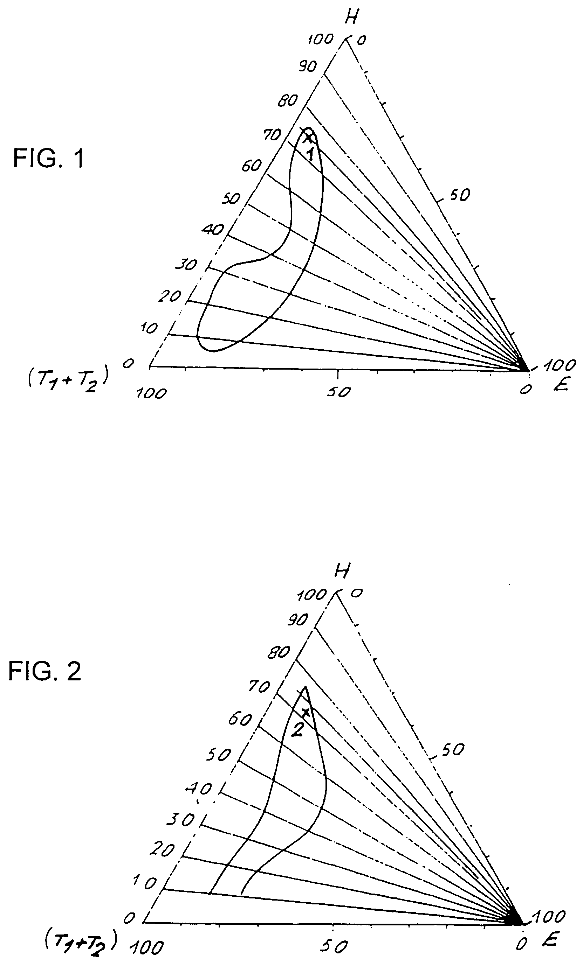

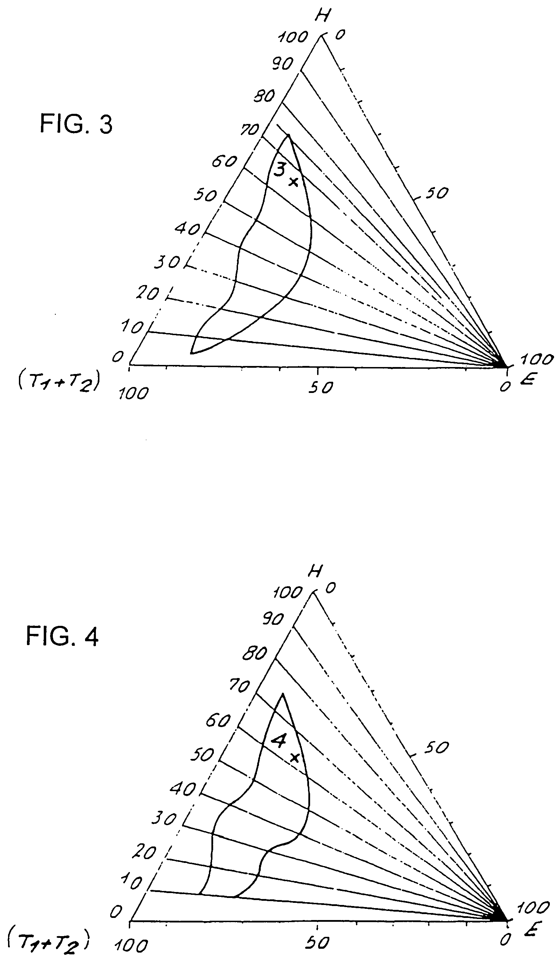

Les exemples 1 à 4 correspondent aux diagrammes ternaires des figures 1 à 4 respectivement.

| Constituants | Exemple 1 | Exemple 2 | Exemple 3 | Exemple 4 |
|---|---|---|---|---|
| PPG-15 stéaryl éther (H) | 70 % | 64 % | 57 % | 50 % |
| Laurate de PEG-400 / laurate de glycéryle(T₁) | 11,5 % | 16 % | 21 % | 24 % |
| Laurate de polyglycéryle-6 (T₂) | 11,5 % | 8 % | 7 % | 6 % |
| Eau (E) | 7 % | 12 % | 15 % | 20 % |
| Rapport T₁/T₂ | 1 | 2 | 3 | 4 |

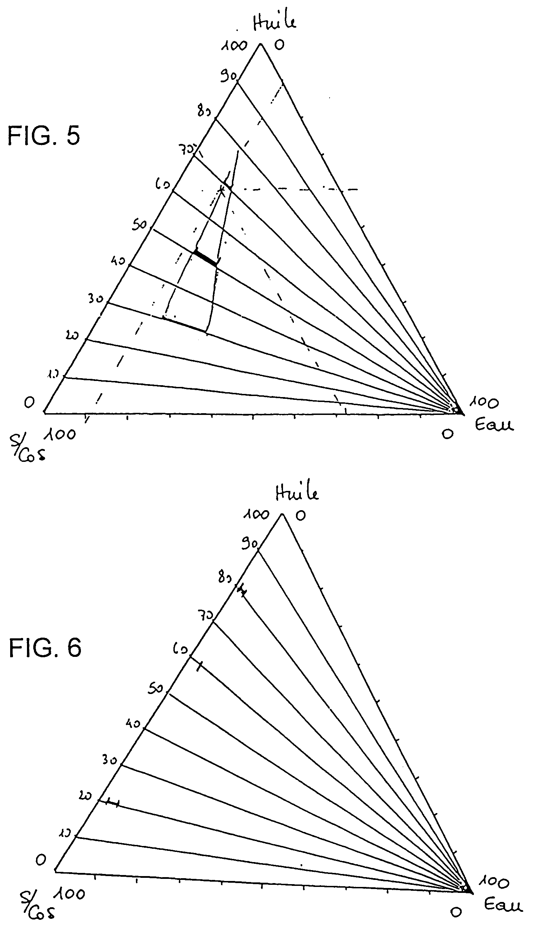

Par ailleurs, les figures 5 et 6 représentent les diagrammes ternaires obtenus avec une composition selon l'invention (figure 5) et avec une composition de l'état de la technique (figure 6). Dans les deux cas, on a utilisé un système émulsionnant/coémulsionnant (S/Cos) comportant comme coémulsionnant l'isostéarate de polyglycéryle-6, le rapport émulsionnant/coémulsionnant étant de 1,5.

Pour la figure 5, conformément à l'invention l'émulsionnant est le laurate de polyéthylène glycol 400 et de glycérol, et pour la figure 6, l'émulsionnant est l'isostéarate de polyéthylène glycol 400 et de glycérol.

Ces diagrammes montrent bien qu'en utilisant un laurate comme coester, on peut obtenir une microémulsion dans une large zone alors qu'avec l'isostéarate, il n'est pas possible d'obtenir de microémulsion.

| **Exemple 5** | |
|---|---|
| Diméthicone (DC 200 fluid de Dow Corning) | 19,00 |
| Isostéarate d'isostéaryle | 44.35 |
| Laurate de PEG-400/laurate de glycérol | 23,20 |
| Isostéarate de polyglycéryle-6 | 5,80 |
| Eau | qsp 100 % |

| **Exemple 6** | |
|---|---|
| PPG-15 stéaryl éther (Arlamol E de la société ICI) | 44,1 |
| Diméthicone (DC 200 fluid de Dow Corning) | 18,9 |
| Laurate de PEG-400 / laurate de glycérol | 20,5 |
| Laurate de polyglycéryle-6 | 6,5 |
| Eau | qsp 100 % |

| **Exemple 7** | |
|---|---|
| Isohexadécane (Arlamol HD de la société ICI) | 44,0 |
| Diméthicone (DC 200 fluid de Dow Corning) | 18,8 |
| Laurate de PEG-400 / laurate de glycérol | 13,5 |
| Laurate de polyglycéryle-6 | 13,5 |
| Glycérine | 2,00 |
| Eau | qsp 100 % |

| **Exemple 8** | |
|---|---|
| PPG-15 stéaryl éther (Arlamol E de la société ICI) | 24,1 |
| Isohexadécane (Arlamol HD de la société ICI) | 20,0 |
| Diméthicone (DC 200 fluid de Dow Corning) | 18,9 |
| Laurate de PEG-400 / laurate de glycérol | 13,5 |
| Laurate de polyglycéryle-6 | 13,5 |
| Eau | qsp 100 % |

Les compositions obtenues dans les exemples 1 à 8 ont l'aspect d'un fluide transparent, ne présentant pas d'amertume et pouvant être utilisé sans gêne pour le visage et notamment autour de la bouche. Ces compositions sont des fluides de base dans lesquels peuvent être introduits un ou plusieurs actifs cités précédemment en vue d'un traitement spécifique.

| **Exemple 9 : fluide amincissant** | |
|---|---|
| Laurate de PEG-400 / laurate de glycérol | 26,5 |
| Isostéarate de polyglycéryle-6 | 10,5 |
| Ethanol | 4,0 |
| Biorubine (Schmidt-Jourdan) (amincissant) | 10,0 |
| Théophylline (amincissant) | 0,3 |
| Fibrastil (Sederma) (amincissant) | 8,0 |
| Monococoate d'éthyl-2 hexyle | 7,0 |
| Conservateur | 0,1 |
| Parfum | 0,2 |
| Eau | qsp 100 % |

| **Exemple 10 : fluide amincissant** | |
|---|---|
| PPG-15 stéaryl éther (Arlamol E de la société ICI) | 23,1 |
| Isohexadécane (Arlamol HD de la société ICI) | 20,0 |
| Diméthicone (DC 200 fluid de Dow Corning) | 17,9 |
| Oléat d'origan (tonique) | 2,0 |
| Laurate de PEG-400 / laurate de glycérol | 13,5 |
| Isostéarate de polyglycéryle-6 | 13,5 |
| Parfum | 0,2 |
| Caféine | 1,5 |
| Eau | qsp100 % |

| **Exemple 11 : fluide anti-rides** | |
|---|---|
| Laurate de PEG-400 / laurate de glycérol | 17,1 |
| Isostéarate de polyglycéryle-6 | 11,5 |
| Recopier System Lipo (Sederma) (nutritif) | 15,0 |
| Palmitate d'isopropyle | 43,6 |
| Acétate de vitamine E | 0,1 |
| Unitrienol T-27 (Lipo) (anti-rides) | 1,2 |
| Conservateur | 0,2 |
| Collagène marin (restructurant) | 2,5 |
| Glycérine (hydratant) | 1,0 |
| Parfum | 0,2 |
| Eau | qsp 100 % |

| **Exemple 12 : fluide restructurant** | |
|---|---|
| Laurate de PEG-400 / laurate de glycérol | 23,2 |
| Laurate de polyglycéryle-6 | 5,8 |
| Isostéarate d'isostéaryle | 42,3 |
| Diméthicone (DC 200 fluid de Dow Corning) | 19,0 |
| Keratoplast (Vevy) (restructurant) | 2,0 |
| AFR LS (Sériobiologiques) (anti-rides) | 2,0 |
| Hydroviton (Dragoco) (hydratant) | 3,0 |
| Acide salicylique (restructurant) | 0,2 |
| Conservateur | 0,2 |
| Parfum | 0,2 |
| Eau | qsp 100 % |

## Revendications

1. Microémulsion cosmétique et/ou dermatologique comportant comme émulsionnant un coester de polyéthylèneglycol et de glycérol avec un acide gras et comme coémulsionnant un ester de polyglycérol avec un acide gras, caractérisée en ce qu'elle comprend au moins 30 % en poids d'huile par rapport au poids total de la microémulsion et en ce que le coester est un laurate de polyéthylèneglycol / laurate de glycérol.

2. Microémulsion cosmétique et/ou dermatologique selon la revendication 1, caractérisée en ce que le coémulsionnant est un isostéarate de polyglycéryle ou un laurate de polyglycéryle.

3. Microémulsion cosmétique et/ou dermatologique selon la revendication 1 ou 2, caractérisée en ce qu'elle contient de 10 % à 60 % en poids d'émulsionnant par rapport au poids total de la microémulsion.

4. Microémulsion cosmétique et/ou dermatologique selon l'une quelconque des revendications 1 à 3, caractérisée en ce qu'elle contient de 4 à 40 % en poids de coémulsionnant par rapport au poids total de la microémulsion.

5. Microémulsion cosmétique et/ou dermatologique selon l'une quelconque des revendications 1 à 4, caractérisée en ce que la phase huileuse représente jusqu'à 75 % en poids de la composition par rapport au poids total de la microémulsion.

6. Microémulsion cosmétique et/ou dermatologique selon l'une quelconque des revendications 1 à 5, caractérisée en ce qu'elle contient un actif hydratant.

7. Utilisation de la microémulsion selon l'une quelconque des revendications 1 à 6 pour un traitement cosmétique de la peau, des ongles et/ou des cheveux.

8. Utilisation cosmétique de la microémulsion selon l'une quelconque des revendications 1 à 6 pour hydrater, nourrir, protéger, raffermir, éliminer les rides et/ou les ridules, amincir et/ou dépigmenter.

9. Utilisation de la microémulsion selon l'une quelconque des revendications 1 à 6 pour la préparation d'une composition dermatologique pour hydrater, nourrir, protéger, raffermir, éliminer les rides et/ou les ridules, amincir et/ou dépigmenter.

10. Utilisation de la microémulsion selon l'une quelconque des revendications 1 à 6 pour la préparation d'une composition destinée au traitement des maladies de la peau et/ou des ongles.

11. Procédé de traitement cosmétique de la peau, des ongles et/ou des cheveux caractérisé en ce qu'il consiste à appliquer sur la peau, les ongles et/ou les cheveux une microémulsion selon l'une quelconque des revendications 1 à 6.

## Claims

1. Cosmetic and/or dermatological microemulsion containing, as emulsifying agent, a fatty acid coester of polyethylene glycol and of glycerol, and, as co-emulsifying agent, a fatty acid ester of polyglycerol, characterized in that it comprises at least 30% by weight of oil relative to the total weight of the microemulsion and in that the coester is a polyethylene glycol laurate/glyceryl laurate.

2. Cosmetic and/or dermatological microemulsion according to Claim 1, characterized in that the co-emulsifying agent is a polyglyceryl isostearate or a polyglyceryl laurate.

3. Cosmetic and/or dermatological microemulsion according to Claim 1 or 2, characterized in that it contains from 10% to 60% by weight of emulsifying agent relative to the total weight of the microemulsion.

4. Cosmetic and/or dermatological microemulsion according to any one of Claims 1 to 3, characterized in that it contains from 4 to 40% by weight of co-emulsifying agent relative to the total weight of the microemulsion.

5. Cosmetic and/or dermatological microemulsion according to any one of Claims 1 to 4, characterized in that the oily phase represents up to 75% by weight of the composition relative to the total weight of the microemulsion.

6. Cosmetic and/or dermatological microemulsion according to any one of Claims 1 to 5, characterized in that it contains a moisturizing active agent.

7. Use of the microemulsion according to any one of Claims 1 to 6 for a cosmetic treatment of the skin, the nails and/or the hair.

8. Cosmetic use of the microemulsion according to any one of Claims 1 to 6 to moisturize, nourish, protect, firm, remove wrinkles and/or fine lines, slim and/or depigment.

9. Use of the microemulsion according to any one of Claims 1 to 6 for the preparation of a dermatological composition to moisturize, nourish, protect, firm, remove wrinkles and/or fine lines, slim and/or depigment.

10. Use of the microemulsion according to any one of Claims 1 to 6 for the preparation of a composition intended for the treatment of diseases of the skin and/or of the nails.

11. Cosmetic treatment process for the skin, the nails and/or the hair, characterized in that it consists in applying to the skin, the nails and/or the hair a microemulsion according to any one of Claims 1 to 6.

## Patentansprüche

1. Kosmetische und/oder dermatologische Mikroemulsion, die als Emulgator einen Coester von Polyethylenglykol und Glycerin mit einer Fettsäure und als Coemulgator einen Ester von Polyglycerin mit einer Fettsäure enthält, dadurch gekennzeichnet, daß sie mindestens 30 Gew.-% Öl, bezogen auf das Gesamtgewicht der Mikroemulsion, enthält, und der Coester ein Laurat von Polyethylenglykol / Laurat von Glycerin ist.

2. Kosmetische und/oder dermatologische Mikroemulsion nach Anspruch 1, dadurch gekennzeichnet, daß der Coemulgator ein Polyglycerylisostearat oder ein Polyglyceryllaurat ist.

3. Kosmetische und/oder dermatologische Mikroemulsion nach Anspruch 1 oder 2, dadurch gekennzeichnet, daß sie 10 bis 60 Gew.-% Emulgator, bezogen auf das Gesamtgewicht der Mikroemulsion, enthält.

4. Kosmetische und/oder dermatologische Mikroemulsion nach einem der Ansprüche 1 bis 3, dadurch gekennzeichnet, daß sie 4 bis 40 Gew.-% Coemulgator, bezogen auf das Gesamtgewicht der Mikroemulsion, enthält.

5. Kosmetische und/oder dermatologische Mikroemulsion nach einem der Ansprüche 1 bis 4, dadurch gekennzeichnet, daß die Ölphase bis zu 75 Gew.-% der Zusammensetzung, bezogen auf das Gesamtgewicht der Mikroemulsion, ausmacht.

6. Kosmetische und/oder dermatologische Mikroemulsion nach einem der Ansprüche 1 bis 5, dadurch gekennzeichnet, daß sie einen hydratisierenden Wirkstoff enthält.

7. Verwendung der Mikroemulsion nach einem der Ansprüche 1 bis 6 zur kosmetischen Behandlung der Haut, der Nägel und/oder dem Haar.

8. Kosmetische Verwendung der Mikroemulsion nach einem der Ansprüche 1 bis 6, um zu hydratisieren, zu nähren, zu schützen, zu straffen, Falten und/oder Fältchen zu beseitigen, schlanker zu machen und/oder zu depigmentieren.

9. Verwendung der Mikroemulsion nach einem der Ansprüche 1 bis 6 zur Herstellung einer dermatologischen Zusammensetzung, um zu hydratisieren, zu nähren, zu schützen, zu straffen, Falten und/oder Fältchen zu entfernen, schlanker zu machen und/oder zu depigmentieren.

10. Verwendung der Mikroemulsion nach einem der Ansprüche 1 bis 6 zur Herstellung einer Zusammensetzung, die zur Behandlung von Krankheiten der Haut und/oder der Nägel bestimmt ist.

11. Verfahren zur kosmetischen Behandlung der Haut, der Nägel und/oder dem Haar, das dadurch gekennzeichnet ist, daß es darin besteht, auf die Haut, die Nägel und/oder das Haar eine Mikroemulsion nach einem der Ansprüche 1 bis 6 aufzutragen.
